# EUROPEAN PATENT SPECIFICATION

(11) **EP 0 238 264 B1**
(45) Date of publication and mention of the grant of the patent: **29.11.1995**
(21) Application number: 87302157.0
(22) Date of filing: 13.03.1987
(51) Int. Cl.: A61B 17/08

(54) **Improved surgical fastening systems made from polymeric materials**
Chirurgisches Klemmsystem aus Polymerwerkstoff
Système de fermeture chirurgicale en matériaux de fermeture chirurgical polymères

(30) Priority: 14.03.1986 US 839407
(43) Date of publication of application: 23.09.1987
(73) Proprietor: ETHICON INC., Somerville New Jersey 08876 (US)
(72) Inventor: Bedi, James J., Stockton, NJ 08558 (US); Gaterud, Mark Turner, Annandale, NJ 08801 (US); Golden, Donald Max, Cherry Hill, NJ 08034 (US); Miksza, Anthony Stanley, Jersey City, NJ 07305 (US)
(74) Representative: Jones, Alan John

(56) References cited:
- EP-A- 0 129 442
- EP-A- 0 136 949
- US-A- 4 534 352

## Description

The present invention relates to surgical fastening systems for closing wounds and more particularly to surgical fastening systems made from polymeric materials for use in closing internal wounds in humans or animals.

### Background of the Invention

For many years surgical wounds and other internal and skin wounds, have been closed using sutures and needles. In more recent times a number of skin stapling instruments have been developed. These instruments apply a series of metal staples to an external wound; that is, a skin wound, to close the wound. In many instances, such skin stapling instruments have replaced the suturing of such wounds. Also, in more recent times instruments have been developed to apply metal fasteners such as staples internally to close internal wounds during a surgical procedure. The primary advantages of the instruments which apply staples to wounds is that they greatly reduce the time required to close the wound and present minimum traumatic effects to improve healing of tissue. Both factors lead to reduced blood loss and improved patient care. Thus, these instruments have considerable medical benefits and economic benefits in surgical procedures. A major disadvantage of closing wounds with metallic staples are that very often the patient requires subsequent diagnostic procedures such as x-rays, cat scanning, and the like, and the metal staples often disrupt such diagnostic procedures. To overcome this last drawback, a number of polymeric fastening systems have been developed which use fasteners made from polymeric materials placed by a suitable instrument to close the wound. Examples of such fastening systems are more fully disclosed in U.S. Patents 4,060,089, 4,532,927; 4,532,926; and 4,513,746.

In European patent application No. 84401937.2, Publication No. 0136949, there is disclosed a surgical fastening system made from polymeric materials which has a primary use of closing internal wounds. The system comprises a two-piece fastening member that is a staple which penetrates the tissue to be closed and a retainer or receiver which interlocks with that staple once it has penetrated the tissue to maintain the wound closed. In this patent, the retainers are connected to one another by yieldable links, that is, flexible or frangible links. The links are used to maintain the receivers together to assist in loading instruments with the receivers.

While such polymeric fastening systems may be made from many different polymer materials, it is desirable that the fasteners be made from absorbable polymeric materials; that is, material that will be hydrolyzed or absorbed by human tissue. By making the fasteners of such absorbable materials, once placed and once the tissue is joined, the material is absorbed by the human body and, hence, no foreign material remains in the human body which may be disruptive and cause complications. It is important to make the fasteners of material that will have sufficient strength to maintain its integrity after it has been placed to hold the tissue together for a sufficient period of time to allow the tissue to heal before the material starts to lose its strength and be absorbed.

What we have discovered is a specific configuration of a fastening system comprising staples and receivers which not only provides for ease and loading of the instruments with the fasteners and, hence, the desired economics, but also has sufficient strength to maintain the desired configuration until the tissue has healed. It is a further object of the present invention to provide a configuration which will move or flex with the tissue that has been joined yet will maintain its configuration and aid in the healing or joining of the tissue. We have discovered that by joining the receivers in a two-piece polymeric system in a specific manner, not only do we get the economic benefits in manufacture and the medical benefits in reducing the time for the surgical procedure, but unexpectedly we obtain more desirable healing characteristics when using our new fastening system to join wounds.

### Summary of the Present Invention

Instruments used in the internal fastening of wounds generally apply at least two parallel rows of staples. When the instrument is to apply fasteners, it will apply at least a pair of parallel rows of staples and a pair of parallel rows of receivers. The staples each comprise a pair of legs connected at one end by a link with the free end of the legs of the staples being sharpened or otherwise adapted to penetrate the tissue. The staple is applied to the tissue by penetrating the tissue with the legs so that the linking member lies adjacent the tissue. A receiver, generally a member which has a pair of apertures spaced so that the legs will fit therethrough, is placed on the opposite side of the tissue and the apertures or openings in the receiver engage with the legs of the staple to lock the staple and receiver together and join the tissue. Hence, the instrument is carrying at least two parallel row of staples in one jaw of the instrument which is placed on one side of the tissue and at least two parallel rows of receivers in the opposite jaw of the instrument placed on the other side of the tissue to be joined. The tissue to be joined is placed between the jaws of the instrument, the jaws are brought together, and the staples fired so that the legs penetrate the tissue and engage the openings in the receivers and then the staples and receivers are released from the instrument. In the fastening system shown in EP-A-0 136 949, the receivers are connected to two receivers in an adjacent row by flexible linkages capable of maintaining the integrity of the receivers. In the system of the present invention, each connection between two adjacent receivers comprises a plurality of parallel linkages between the receivers. The linkages are sufficiently flexible and strong to maintain the receivers connected during both the application of the fastening system and the use of the fastening system to join and hold the tissue. In a preferred embodiment of the present invention the area between the openings of a receiver is depressed or recessed. The recesses allow for excess tissue and prevent strangulation of the tissue. This configuration produces good hemostasis while allowing blood to be supplied to the tissue to support tissue life and prevent necrosis. As previously mentioned, it is critical to the present invention that the receivers be joined or held together until the wound has healed. By ensuring that the receivers are held together while the tissue is healing, improved hemostasis is obtained and prevention of wandering of the fasteners is eliminated. It is desired that there be some flexibility in the connected receivers so that if the patient moves and the tissue being joined is moved slightly, the fastening system will move or flex with the tissue and the desired hemostasis maintained. By joining a receiver to two adjacent receivers with at least three parallel linkages at each juncture, we have been able to obtain the desired flexibility in the final closure and maintain good hemostasis throughout the healing process. Furthermore, the receiver being made from absorbable materials, as it is being absorbed by the human body it tends to lose strength and unless adequately and correctly joined may break apart during the healing process which is undesirable. By joining each receiver to at least two receivers and by having each juncture at least three parallel linkages even if one, or two, or three linkages break during the healing process, there is still sufficient connection to maintain the desired hemostasis throughout the healing operation. Furthermore, even if stresses are placed on the wound sufficient to break a couple of the linkages, there are still sufficient linkages to maintain the desired configuration of the fastening system and adequate hemostasis throughout the wound healing process.

### Brief Description of the Drawings

The invention will be more fully described in conjunction with the accompanying drawings wherein:
Figure 1 is a perspective view of the fastening system of the present invention;
Figure 2 is a front view of a staple member of the fastening system of the present invention;
Figure 3 is an end view of the staple shown in Figure 2;
Figure 4 is a top view of a receiver used in the fastening system of the present invention;
Figure 5 is a cross-sectional view taken along line 5-5 of Figure 4;
Figure 6 is another cross-sectional view taken along line 6-6 of Figure 4;
Figure 7 is a top view of a plurality of connected receivers of the fastening system of the present invention;
Figure 8 is an end view of the two lines of receivers shown in Figure 7; and
Figure 9 is a perspective view of the new fastening system in place joining tissue.

### Detailed Description of the Drawings

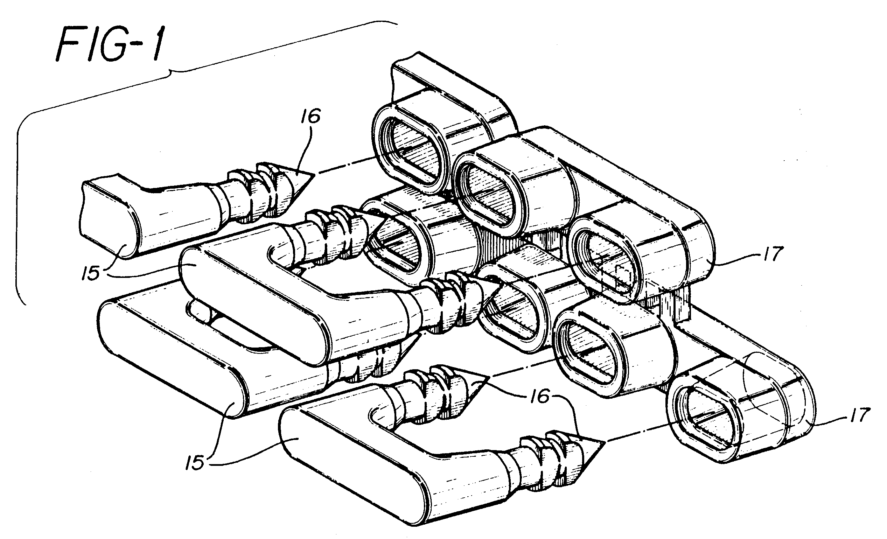

Referring to Figure 1 there is shown a portion of a fastening system of the present invention in a perspective view. The fastening system comprises two parallel rows of individual staples 15. The legs 16 of the staples are designed to penetrate the tissue to be joined and lock or join therein to a plurality of receivers 17. The receivers comprise two parallel rows of individual receivers with each receiver being connected to two receivers in an adjacent row and with each connection between two adjacent receivers comprising a plurality of parallel linkages.

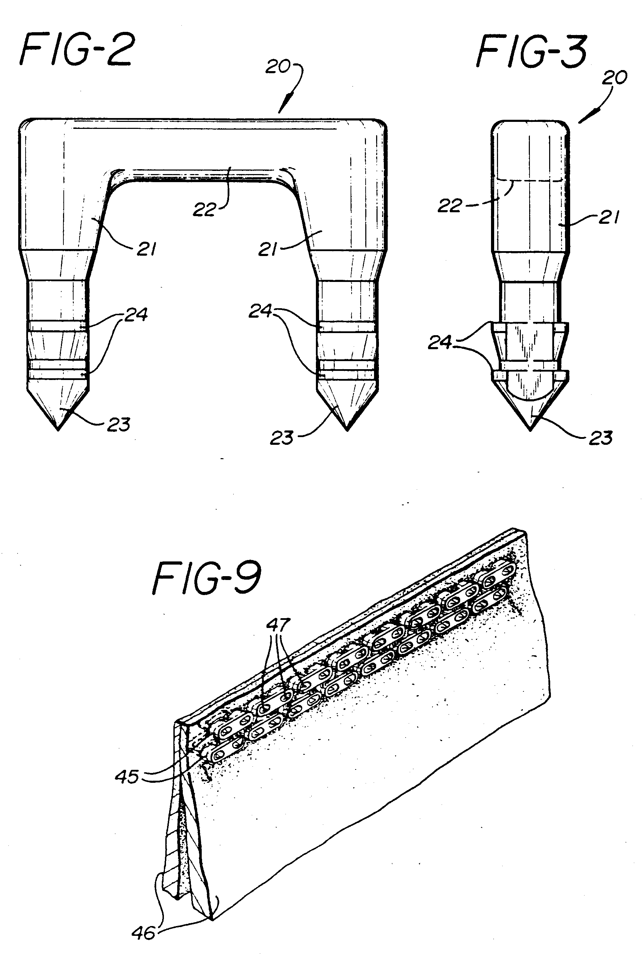

As more clearly shown in Figures 2 and 3, each staple 20 comprises a pair of legs 21. The legs are joined at one end by a suitable linkage 22. In use, the legs penetrate the tissue to be joined, and the linkage rests on the surface of one side of the tissue being joined. Each leg has a free end 23 which in this embodiment is pointed or sharpened to assist in penetrating the tissue. Also disposed along the free ends of the legs are suitable means 24 for locking the legs of the staple with the receiver. In this embodiment, there are two locking means to adjust the distance between the staple and the receiver depending on the thickness of the tissue to be joined. The locking means comprise oppositely disposed ledges which are spaced along the leg of the staple from the pointed end. As will be discussed, these ledges interlock or catch with oppositely disposed ledges in the receiver.

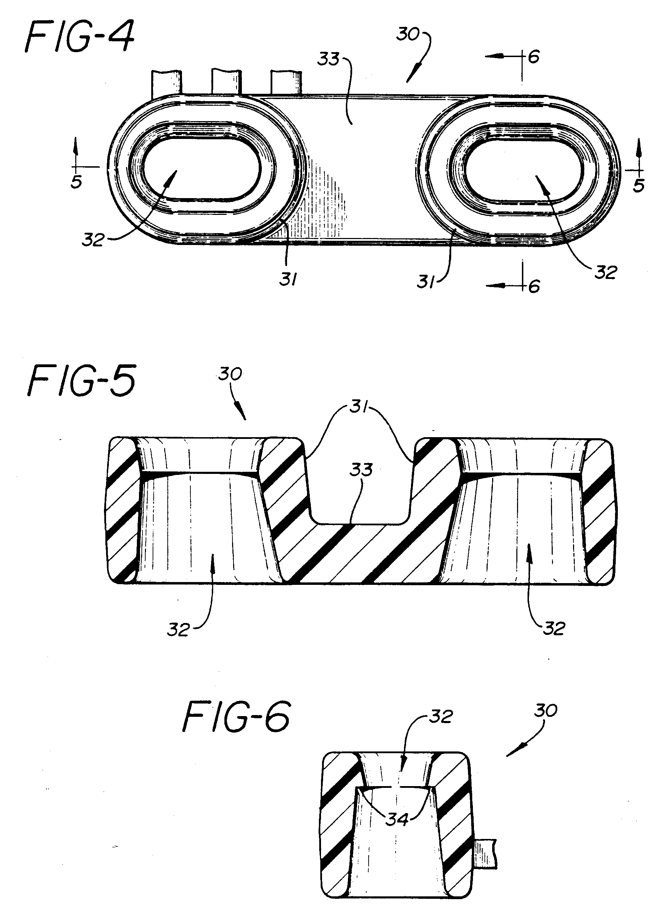

Individual receivers are more clearly depicted in Figures 4, 5, and 6. Each receiver 30 comprises a body member 31 and disposed in that body member are two openings 32. The center point of each opening coincides or is spaced from the center point of the other opening by the distance between the center lines of the staple legs. As shown in Figure 5, it is preferred that the center portion 33 of the body be depressed to allow the fastening system to be used with a broader range of tissue thickness. By shaping the openings as shown, that is in an oval manner, the alignment of the staple legs and the openings is not as critical and if the staple legs tend to bend or get off line slightly when penetrating the tissue, they still will engage the oval openings of the receiver. As shown in Figure 6, immediately below the opening is a ledge 34 and once the staple leg penetrates the tissue the appropriate ledges on the staple legs will be engaged by the ledge in the opening to lock the staple leg therewith. If thick tissue is being joined, the ledges closest to the tip of the leg of the staple engage the ledge in the opening; whereas, if thinner tissue is being joined, the ledges on the staple leg disposed furtherest from the staple leg engage the ledge in the opening of the receiver.

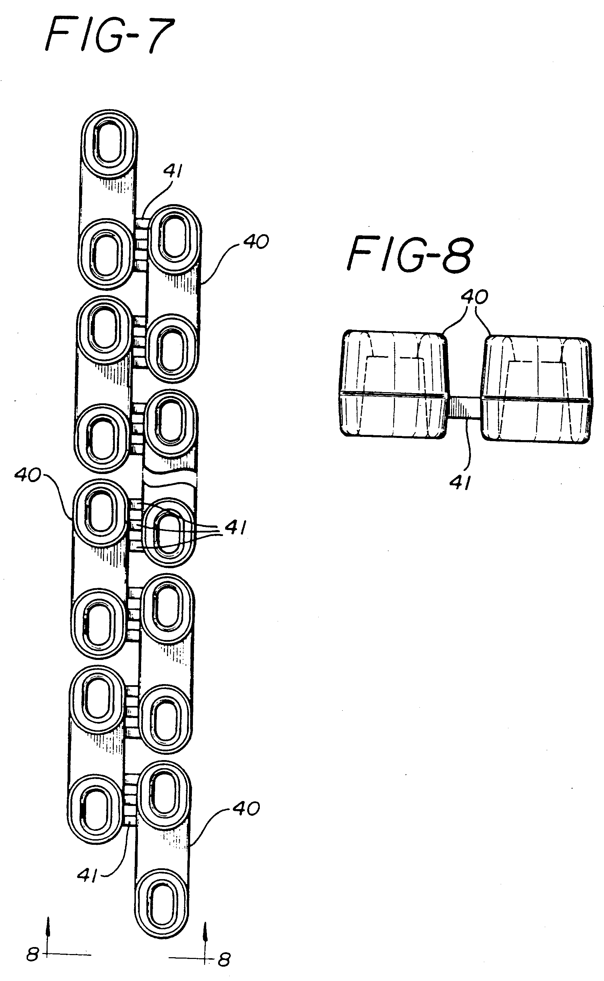

As shown in Figures 7 and 8, there are two parallel rows of receivers. Each receiver 40 is connected to at least two adjacent receivers with the exception, of course, of the last receiver at one end of each row. Each connection is accomplished by a plurality of parallel linkages 41. As previously mentioned, it is important that each receiver be connected to two adjacent receivers and that each connection be a plural connection. This configuration of connections provides the desired integrity, rigidity and flexibility in the receiver to provide excellent hemostasis with our improved fastening system, especially when the system is made from absorbable polymer.

Referring to Figure 9 there is shown the parallel rows of receivers 45 in place on one side of the tissue 46 being joined. The legs 47 of the staples on the opposite side of the tissue are locked in the openings of the receivers to hold the tissue together.

As shown in Figure 8, the linkages are disposed from the top of the receivers and are closer to the bottom of the receivers to allow for excess tissue to overlap and fill up that area if desired.

The fastening systems of the present invention may be made from any of the absorbable polymeric materials. Examples of suitable polymers are the polymers and copolymers of lactide, glycolide, dioxanone, and the like.

## Claims

1. A surgical fastening system for joining tissue (46), said system comprising a plurality of parallel rows of staples (15, 20) and a plurality of parallel rows of receivers (17, 30, 40, 45), each of said staples (15, 20) comprising a pair of legs (16, 21), said legs (16, 21) being joined at one end thereof by a linking member (22), the opposite end of each leg (16, 21) being adapted to penetrate tissue (46) so the linking member (22) lies adjacent the tissue (46) penetrated by said legs (16, 21), each receiver (17, 30, 40, 45) having a body portion (31) and a plurality of openings (32) disposed in said body portion (31), said openings (32) being located so as to engage the legs (16, 21) of the staple (15, 20) after said legs (16, 21) have penetrated the tissue (46) so that the receiver (17, 30, 40, 45) lies on the opposite side of the tissue (46) to be joined, and means (24, 34) on said legs (16, 21) and on said receivers (17, 30, 40, 45) for interlocking the staple (15, 20) and the reciever (17, 30, 40, 45) together, each receiver (17, 30, 40, 45) being connected to two receivers (17, 30, 40, 45) in an adjacent row by flexible linkages (41) capable of maintaining the integrity of the recievers (17, 30, 40, 45), characterized by each connection between two adjacent receivers (17, 30, 40, 45) comprising a plurality of parallel linkages (41) which are sufficiently flexible and strong to maintain the receivers (17, 30, 40, 45) connected during both the application of the fastening system when joining the tissue (46) and the use of the fastening system to hold the tissue (46) until such tissue (46) has healed.

2. A surgical fastening system according to Claim 1 wherein the openings (32) in the receiver (17, 30, 40, 45) each have an oval shape.

3. A surgical fastening system according to Claim 1 wherein each connection between receivers (17, 30, 40, 45) comprises three parallel linkages (41).

## Patentansprüche

1. Chirurgisches Verbindungssystem zum Verbinden von Gewebe (46), wobei das System eine Anzahl von parallelen Reihen von Klammern (15, 20) und eine Anzahl von parallelen Reihen von Aufnahmeelementen (17, 30, 40, 45) umfaßt, wobei jede der Klammern (15, 20) zwei Schenkel (16, 21) aufweist, die an ihrem einen Ende durch ein Verbindungselement (22) verbunden sind, während die gegenüberliegenden Enden jedes Schenkels (16, 21) dafür vorgesehen sind, Gewebe (46) so zu durchdringen, daß das Verbindungselement (22) an dem von den Schenkeln (16, 21) durchdrungenen Gewebe (46) anliegt, wobei jedes Aufnahmeelement (17, 30, 40, 45) einen Hauptabschnitt (31) und eine Anzahl von Öffnungen (32) im Hauptabschnitt (31) aufweist, wobei die Öffnungen (32) so angeordnet sind, daß sie mit den Schenkeln (16, 21) der Klammern (15, 20) in Eingriff kommen, nachdem die Schenkel (16, 21) das Gewebe (46) durchdrungen haben, so daß das Aufnahmeelement (17, 30, 40, 45) auf der gegenüberliegenden Seite des zu verbindenden Gewebes (46) liegt, und mit Einrichtungen (24, 34) an den Schenkeln (16, 21) und an den Aufnahmeelementen (17, 30, 40, 45) zum Ineinandergreifen der Klammern (15, 20) und der Aufnahmeelemente (17, 30, 40, 45), wobei jedes Aufnahmeelement (17, 30, 40, 45) durch flexible Zwischenstücke (41), die in der Lage sind, die Integrität der Aufnahmeelemente (17, 30, 40, 45) aufrecht zu erhalten, mit zwei Aufnahmeelementen (17, 30, 40, 45) in der angrenzenden Reihe verbunden ist, dadurch **gekennzeichnet**, daß jede Verbindung zwischen zwei benachbarten Aufnahmeelementen (17, 30, 40, 45) eine Anzahl von parallelen Zwischenstücken (41) umfaßt, die ausreichend flexibel und kräftig sind, um die Aufnahmeelemente (17, 30, 40, 45) sowohl während des Anlegens des Verbindungssystems beim Verbinden von Gewebe (46) als auch bei der Verwendung des Verbindungssystems zum Zusammenhalten des Gewebes (46), bis das Gewebe verheilt ist, in Verbindung zu halten.

2. Chirurgisches Verbindungssystem nach Anspruch 1, wobei die Öffnungen (32) in den Aufnahmeelementen (17, 30, 40, 45) jeweils eine ovale Form haben.

3. Chirurgisches Verbindungssystem nach Anspruch 1, wobei die Verbindung zwischen den Aufnahmeelementen (17, 30, 40, 45) drei parallele Zwischenstücke (41) umfaßt.

## Revendications

1. Système de fixation chirurgicale destiné à réunir des tissus (46), ledit système comprenant une pluralité de rangées parallèles d'agrafes (15, 20) et une pluralité de rangées parallèles de réceptacles (17, 30, 40, 45) chacune desdites agrafes (15, 20) comprenant une paire de branches (16, 21), lesdites branches (16, 21) étant réunies à une de leurs extrémités par un élément de liaison (22) l'extrémité opposée de chaque branche (16, 21) étant adaptée pour traverser les tissus (46) de manière que l'élément de liaison (22) soit adjacent aux tissus (46) traversés par lesdites branches (16, 21), chaque nréceptacle (17, 30, 40, 45) ayant une portion corps (31) et une pluralité d'ouvertures (32) disposées dans ladite portion corps (31), lesdites ouvertures (32) étant positionnées de façon à coopérer avec les branches (16, 21) de l'agrafe (15, 20) après que lesdites branches (16, 21) ont traversé les tissus (46), de sorte que le réceptacle (17, 30, 40, 45) se trouve de l'autre côté des tissus à réunir, et des moyens (24, 34) prévus sur lesdites branches (16, 21) et sur lesdits réceptacles (17, 30, 45) pour verrouiller l'agrafe (15, 20) et le réceptacle (17, 30, 40, 45) l'un à l'autre, chaque réceptacle (17, 30, 40, 45) étant relié à deux réceptacles (17, 30, 40, 45) situés dans une rangée adjacente par des liens flexibles (41) capables de maintenir l'intégrité des réceptacles (17, 30, 40, 45), caractérisé en ce que chaque moyen d'assemblage entre deux réceptacles (17, 30, 40, 45) adjacents comprend une pluralité de liaisons parallèles (41) qui sont suffisamment flexibles et solides pour maintenir les réceptacles (17, 30, 40, 45) assemblés aussi bien pendant l'application du système de fixation, au moment de la réunion des tissus (46) que pendant l'utilisation du système de fixation, pour maintenir les tissus (46) jusqu'à ce que ces tissus (46) soient guéris.

2. Système de fixation chirurgicale selon la revendication 1, dans lequel les ouvertures (32) du réceptacle (17, 30, 40, 45) ont chacune une forme ovale.

3. Système de fixation chirurgicale selon la revendication 1, dans lequel chaque moyen d'assemblage entre réceptacles (17, 30, 40, 45) comprend trois liens parallèles (41).
